# EUROPEAN PATENT SPECIFICATION

(11) **EP 2 347 736 B1**
(45) Date of publication and mention of the grant of the patent: **16.01.2013**
(21) Application number: 10151587.2
(22) Date of filing: 25.01.2010
(51) Int. Cl.: A61F 2/46

(54) **Inserter for locating and impacting an acetabular cup**
Inserter zur Lokalisierung und Zusammenpressung einer Gelenkpfanne
Applicateur pour localiser et agir sur une coupe acétabulaire

(43) Date of publication of application: 27.07.2011
(73) Proprietor: Benoist Girard SAS, 14201 Hérouville-Saint-Clair Cédex (FR)
(72) Inventor: Rushton, Neil, Prof., Cambridge, Cambridgeshire CB2 8AW (GB); Field, Richard E, Tadworth, Surrey KT20 7UB (GB); Auxepaules, Arnaurd, 14750, Saint Aubin sur Mer (FR); Mehats Hibon, Aude, 35400, Saint Malo (FR)
(74) Representative: Bridge-Butler, Jeremy

(56) References cited:
- EP-A1- 0 888 759
- WO-A1-2004/069107
- WO-A2-2008/029102
- DE-A1-102008 047 627
- US-A- 5 171 243

## Description

The present invention relates to an inserter for locating and implanting an acetabular cup and is particularly although not exclusively applicable for use with so-called flexible cups. Previous known inserters are usually only designed for use with a particular size of cup and are not adaptable for use with other types of cups.

The present invention is intended to provide a construction which can be used for either holding a cup whilst it is placed in position and which can also be used for impacting the cup into the acetabulum. The invention is also intended to ensure that the inserter is not used to impact the cup whilst it is still attached to the inserter and is constructed so that if the inserter is impacted with the retaining means in place holding the cup the cup will instantly be released.

The instrument is also intended to compensate for deformation of the cup caused by the cups flexibility.

WO 2004/069107, in the name of Depuy International Limited, discloses an inserter for locating and impacting an acetabular cup which can hold a cup whilst it is placed in position, and which can also be used for impacting the cup into the acetabulum. It comprises a head with radially extending flange portions which are movable between an in use position in which they engage a groove on an associated cup to retain it for location, and a retracted position in which they disengage the cup. A face is provided on the head which engages the rim of the cup, and which allows for an impaction force to be imparted to the cup to force it into the bone recess. However, to release the cup from the head a sliding collar must be axially-displaced into engagement with the flange portions to bias them into the retracted position. Therefore, there is no means to release the cup from the inserter if it is inadvertently impacted with the cup still attached. WO 2004/069 107 is forming the basis for the preamble of claim 1.

According to the present invention an inserter for locating and impacting an acetabular cup which has an inner bearing surface in a prepared acetabulum comprises a handle, an impaction head adapted to receive said cup, retention means for attaching said cup to the impaction head, and actuating means for freeing the retention means from the cup, characterised in that the handle carries an anvil, in which the actuating means is in the handle and is operatively connected to the anvil, in which the retention means extends between the actuating means and the impaction head, and in which impaction of the anvil operates said actuating means to free the retention means from the cup.

Thus, if any attempt is made to use the inserter with the cup attached to it the retention means are immediately released when the anvil is impacted.

In one preferred construction the acetabular cup has an opening in its inner bearing surface at least part of which extends adjacent to or over a substantially central part of the cup and in which in a first position of said actuating means the retention means extends into the opening in the inner bearing surface and engage the cup to hold it in position on the impaction head, and in which said actuating means can be operated to a second position to free said retention means from the cup by impacting the anvil.

With the above arrangement the opening in the inner bearing surface can extend through the wall of the cup to its outer surface to provide an outer rim which can be engaged by the retention means.

Preferably the actuating means can also be operated to free the retaining means independently from the anvil.

The retention means can include a retainer which in the first position of the actuating means is raised and engages the outer rim of the opening in the cup and in which in the second position the actuating means is rotated and lowered relative to its first position and is clear of and lower than the outer rim.

The retainer can be carried on a rotatable and axially movable elongate operating member which extends between the impaction head and the actuating means in the handle.

With this arrangement means for resiliently biasing the actuating means towards the first and second positions can be provided and can be in the form of a compression spring which acts on said rotatable operating member.

The retention means may conveniently include a projecting finger or hook which in the first position of the actuating means extends over or against the side of the opening in the cup and in which in the second position of the actuating means is located within or below the opening in the cup.

The actuating means can include a control member which projects radially from the side of the handle and which is located in a control slot within which it can move angularly to rotate the retention means, said slot being shaped to cause the retention means to also move axially when rotated. Thus with this arrangement the cup can be locked in position or released by operation of the control member.

The slot can have indentations at each of its ends to locate the control member in the first or second position of the actuating means, and the slot is preferably angled towards the end which corresponds to the second position of the actuating means.

With the above arrangements the anvil engages the end of the handle when in the second position and thus, when it is used for impacting the cup, the load is carried through the handle to the impaction head.

In an alternative construction the opening in the inner bearing surface of the cup can be formed with a socket with an inner slot into which the retention means extend to engage and hold the cup in position on the impaction head.

This type of construction can be operated in a similar manner to that described above with regard to constructions in which the opening extends from the inner bearing surface to the outer surface of the cup.

In another alternative construction the retention means may comprise two or more retention pads which can be operated by the actuating means to expand and grip the inner bearing surface of the cup. This construction is particularly adaptable for use with cups which do not have an opening in the inner bearing surface.

The impaction head is arranged so that it can be readily removable from the handle and the invention can therefore include two or more impaction heads of different configurations or size which can be fitted alternatively to the handle.

The invention can be performed in various ways and some embodiments will now be described by way of example and with reference to the accompanying drawings in which:
Figure 1 is an isometric view from above of an acetabular cup with an opening extending between its inner bearing surface and its outer surface and which can be used with the inserter according to the invention;
Figure 2 is an exploded isometric view of the main parts of the inserter according to the invention but not showing the impaction head or the cup with which it is to be used;
Figure 3 is a plan view of the parts shown in Figure 2 assembled ready for use;
Figure 4 is an end view of the inserter shown in Figure 3;
Figure 5 is a side view of part of the handle and anvil of the construction shown in Figure 3;
Figure 6 is a side view of the construction shown in Figures 3 to 5 with the acetabular cup in place and the handle and part of the actuating means being shown in cross-section;
Figure 7 is an isometric view of the inserter shown in Figure 6 with similar parts in cross-section;
Figure 8 is a plan view of part of the handle and operating means and anvil of the construction shown in Figures 6 and 7;
Figure 9 is an enlarged side view of the cup and retention means shown in Figure 7 and which are in the first operating position;
Figure 10 is a side elevation of the construction shown in the previous Figures and in which the actuating means and retention means are in a second position in which the cup is released;
Figure 11 is an isometric view of the anvil and the operating means shown in Figure 10;
Figure 12 is an external view of the handle and anvil when in the position shown in Figure 10;
Figure 13 is an external isometric view of the handle and anvil with the operating means in the second position as shown in Figure 11;
Figure 14 is a side view of the impaction head of an alternative construction and with the impaction head and cup in cross-section;
Figure 15 shows another alternative embodiment of the impaction head which can be used with a cup which does not have an opening between its inner bearing surface and its outer surface; and,
Figure 16 is a plan view of the construction shown in Figure 15 but with the cup removed.

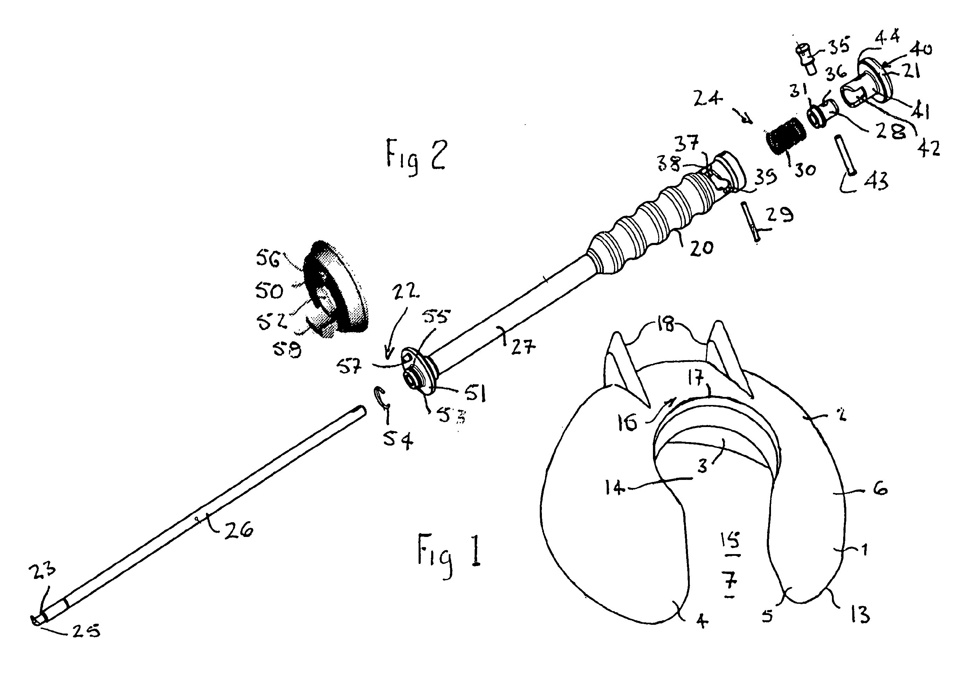

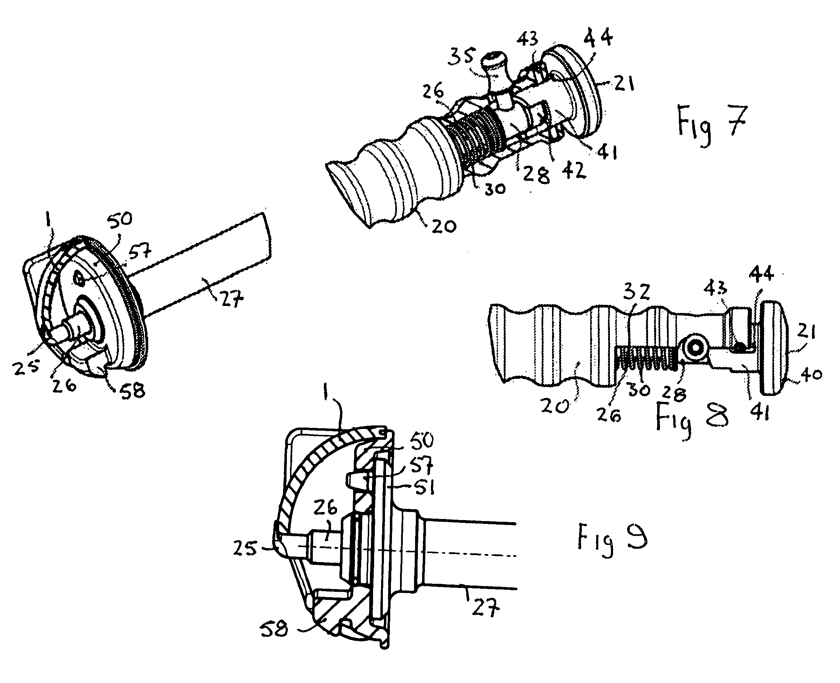

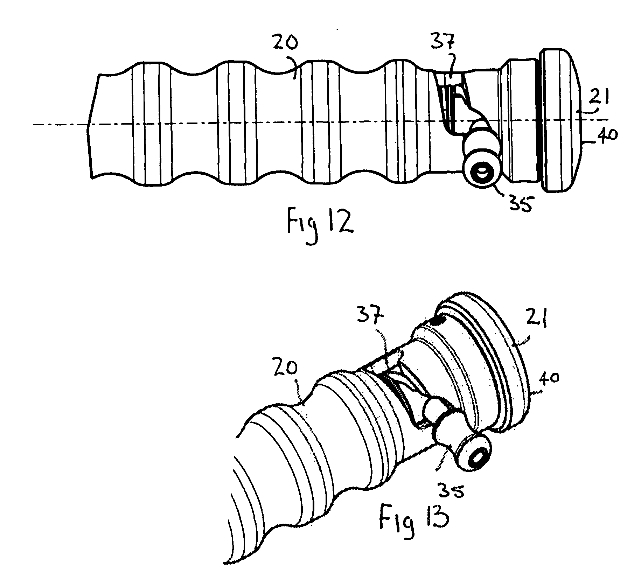

Figure 1 shows a flexible cup with which the construction of an inserter shown in Figures 2 to 13 is intended to be used. The cup 1 is of the type shown in European Patent No. 1 205 160 and US 6 758 864. The cup 1 basically comprises a main portion 2 which has a part-spherical inner bearing surface 3 and there are two independent arms 4 and 5 which extend from the main part 2. The outer surface 6 of the cup is also part-spherical. The arms 4 and 5 are spaced apart to provide a gap or opening 7 between them and the opening breaks out of the cup rim 13.

The main part 14 of the opening 7 is substantially semicircular and has a mouth 15 which provides the interruption in the rim 13 and which is of smaller width than the main part 14 of the opening.

The main part 14 of the opening 7 in the bearing surface 3 which extends adjacent to or over a substantially central part of the cup indicated by reference numeral 16 and extends through the wall of the cup 1 to its outer surface 6 and has an outer rim 17.

The outer surface 2 of the cup carries a pair of projecting fins 18 which extend in spaced apart parallel chordal directions.

The inserter according to the invention for use with the cup shown in Figure 1 comprises a handle 20 carrying an anvil 21, the impaction head 22 adapted to receive the cup 1 and retention means 23 which extend between actuating means 24 in the handle 20 and the impaction head 22, the construction is such that the actuating means 24 are operable to free the retention means 23 from the cup by impacting the anvil 21.

The retention means 23 include a retainer 25 in the shape of a projecting finger or hook which is carried on a rotatable axially movable elongate operating member 26 which extends between the impaction head 22 and the actuating means 24 in the handle 20. The handle 20 has a hollow extension 27 through which the elongate operating member 26 extends and enters a flanged sleeve 28 and is held in position by a rivet 29. The handle 20 has an enlarged bore at its outer end in which the end of the elongate operating member 26 is located and in which is provided a coiled compression spring 30 which surrounds the elongate operating member 26. One end of the spring 30 engages a flange 31 on the flanged sleeve 28 and the other end of the spring engages a shoulder 32 which terminates the enlarged portion of the bore in the handle 20. Thus, the spring 30 resiliently biases the elongate operating member 26 towards the impaction head 22.

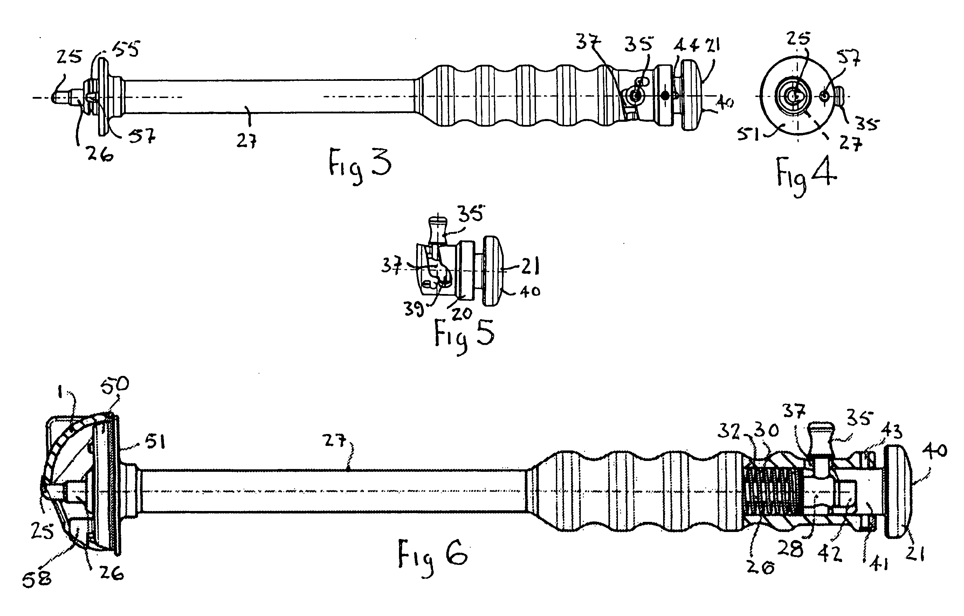

The flanged sleeve 28 carries a control member in the form of a projecting control lever 35 which is located in an opening 36 in the sleeve 28. The operating member 35 projects radially from the flanged sleeve 28 and extends into a circumferentially extending control slot 37 in the handle 20. The control lever 35 can move angularly to rotate the elongate operating member 26, thus rotating the retention means 23. The slot 37 is shaped to cause the operating member 26 to also move axially when rotated and this is achieved by the slot being angled as is most clearly shown in Figure 3. The slot 37 has indentations 38 and 39 at each end to locate the control member in those positions.

The anvil 21 has an impacting surface 40 provided on a mushroom shaped head and a hollow sleeve extension 41 one side of which is provided with a cut-out 42 which is large enough to receive the control lever 35. The anvil 40 is held in position in the enlarged part of the bore 31 of the handle by a pin 43 which is located in appropriate openings in the end of the handle 20. The pin 43 passes through an elongate slot 44 in the extension 41 so that the anvil has limited axial movement in the handle 20.

The impaction head 22 comprises a dished circular compaction plate 50 shaped to fit over a flanged mounting 51 on the hollow extension 27 and has a central opening 52 which fits over a boss 53 and is held in position by a circlip 54 which can engage in a circumferential groove 55. The compaction plate 50 also has a location opening 56 which engages a location boss 57 on the flanged mounting 51 to locate its angular position and it also carries a shaped locator 58 which is shaped to locate in the opening 57 of the cup 1.

As will be seen from Figure 4 the flanged mounting 51 is slightly eccentric in relation to the hollow extension 27 to allow for the retention means 23 to engage the rim 17 of the opening 7 when its lower rim 13 is located on the impaction plate 50, the inner sides of the lower rim 13 engaging the dished side of the plate.

In a first position of the actuating means 24 the control lever 35 is in the position shown in Figures 3 to 9, i.e. the lever 35 is at one end of the circumferentially extending control slot 37 in the indentation 38 and this will be referred to herein as the "first position". In this position the spring 30 is compressed and the retainer 25 is raised away from the compaction plate 50. It is also in a position where it will engage the rim 17 of the opening 7. If the control lever 35 is moved to its second position which is at the other end of the slot 37 it is biased away from the compaction plate 50 and is located in the indentation 39 and this will be referred to herein as the "second position". In this second position the retainer 25 has been located to a position in which it will not engage the rim 17 of the opening 7 and has also been retracted in relation to the compaction plate 50 so that the inserter can be removed from the cup 1.

In order to locate a cup 1 on the inserter the control lever 35 is first moved to the second position and the cup can be placed over the compaction plate 50 with the rim of the cup located on the rim of the compaction plate and the opening 7 located on the shaped locator 58. The cup's angular location on the inserter is therefore determined. The control lever 35 is now moved manually down the inclined control slot 37 to the first position and this causes the elongate operating member 26 to be rotated and, due to the angulation of the slot to move axially thus raising the retainer 25 and simultaneously locating it to engage the rim 17 of the opening 7. In this first position the retainer 25 is extending away from the opening 7 in the cup and engaging the nearest part of the rim 17 of the opening 7 to hold the cup in position against the rim of the compaction plate 50. The spring is compressed and assists in hold the retainer 25 in place.

The inserter can now be used by a surgeon to accurately place the cup into the prepared acetabulum.

The retainer can now be released from the cup by moving the control lever 35 to the other end of the slot 37 to the second position which thus causes the retainer 25 to be rotated out of contact with the rim 17 and simultaneously lowered within the opening 17 in the cup.

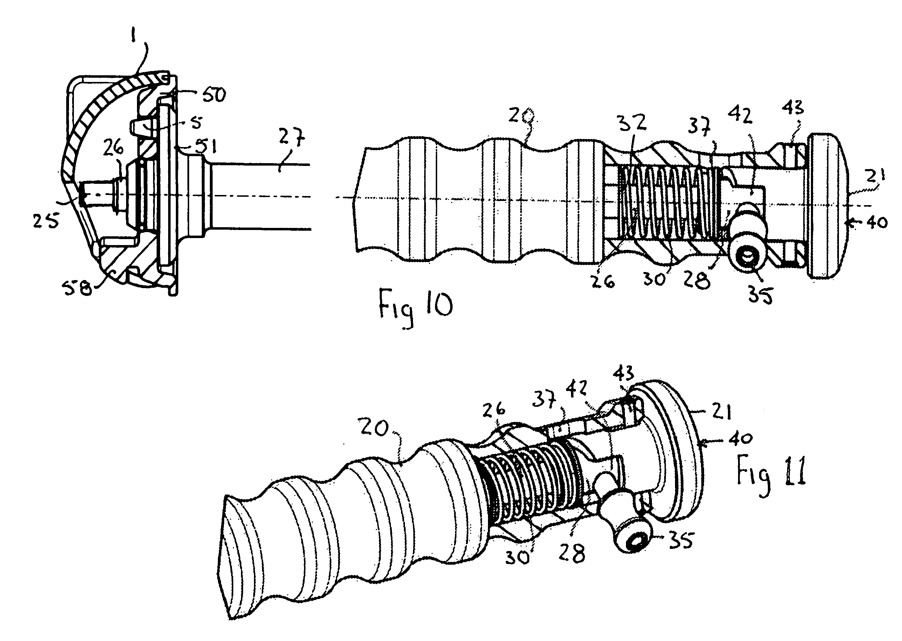

In the first position the axial movement of the elongate operating member 26 causes the anvil 21 to be moved to the right, as shown in the drawings, due to the end of the elongate operating member 26 bearing against the end of the hollow sleeve extension 41. The inserter is constructed so that it is not possible to hammer the cup 1 into place in the prepared acetabulum with the parts in the first position. If the anvil 4 is impacted the control lever 35 is moved from the indentation 38 against the action of the compression spring and the spring then causes the control lever to move along the angled control slot 37 to the second position and to enter indentation 39. This movement to the second position, which is shown in Figures 10 to 13, allows the control lever 35 to enter the cut-out 42 in the extension 41 due to the elongate slot 44 through which the pin 43 passes. The effect of the angular movement therefore frees the retainer 25 and frees the inserter from the cup 1. Due to the control lever 35 now being in the cut-out 42 the anvil 21 now moves downwardly and, as shown in Figures 10 to 13, engages the end of the handle 20. With the parts being in the second position with the anvil 21 engaging the handle 20 the inserter can be used by hammering the anvil 21 to impact the cup into position, the rim of the cup being engaged by the rim of the compaction plate 50.

The invention therefore provides an inserter which is automatically released from the cup if it is impacted with the inserter attached to the cup.

The operating procedure by the surgeon is therefore to first fit the cup with the control lever 25 in the second position, that is, the released position. The lever 25 is then moved to the first position in which the cup is held in place on the inserter. The surgeon can now insert the cup in the acetabulum as required, with the cup located the control lever 25 is now moved to the second position to release the cup and the anvil 21 can be hammered to impact the cup in place.

If the surgeon fails to release the cup before hammering the anvil 21 the cup will be automatically released by the first hammer blow on the anvil 21.

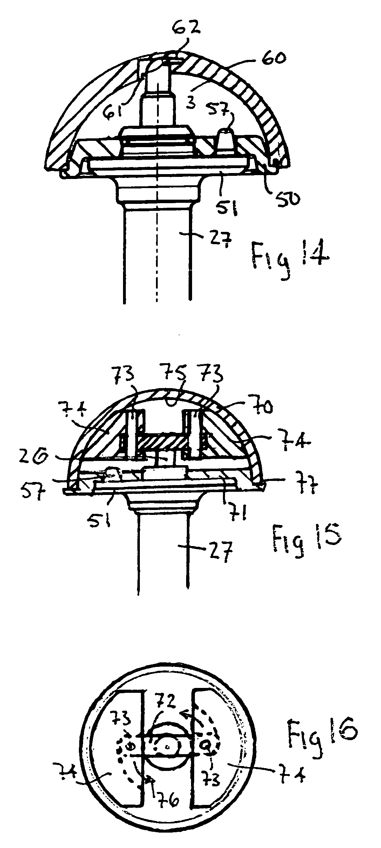

Figure 14 shows an alternative construction which can be used with a different type of cup. In this arrangement the cup 60 has an opening 61 which is formed as a socket with a blank end. The inner end of the socket has a radially projecting slot 62. The general construction of the inserter is similar to that described with regard to the previous embodiment but in this arrangement the shaped locator 58 is omitted from the compaction plate 50. The dimensions of the parts are arranged so that the inserter works in the same way as described with regard to the previous embodiment but in which the retainer 25 does not extend through an opening and onto its outer rim but engages within the radial slot 62 provided in the socket 61.

Figures 15 and 16 show another construction according to the invention in which the inserter is for use with a cup 70 which does not have an opening in its inner bearing surface. In this arrangement the parts are similar to those set forth in the earlier constructions and the same reference numerals are used to indicate similar parts but the retention means 23 are of a different construction. In this arrangement the elongate operating member 26 carries a bar 72 each radially displaced end of which is provided with a pivot pin 73. Each pin acts to pivotally connect two retention pads 74 which provide the retention means. As will be seen from Figure 15 the pads are shaped to engage the inner bearing surface 75 of the cup 70.

In Figures 15 and 16 the bearing pads are shown with the various parts in the first position, i.e. the bearing pads are engaging the inner bearing surface 75 to lock it to the elongate operating member 26. When the parts are moved to the second position the member 72 is located in the direction of the arrows 76 thus retracting the engagement pads 74 from the inner bearing surface 75 and simultaneously lowering them. In this position the inserter can be released from the cup and, as described above, used for hammering the cup into place in the acetabulum, the load being carried through the handle and directly to the rim 77 of the cup.

The bearing parts can be made from any suitable material, for example aluminium or hardened rubber or a synthetic plastics material.

The moving parts of the inserter can also be made of any suitable metal or synthetic plastics material as appropriate.

## Claims

1. An inserter for locating and impacting an acetabular cup (1) which has an inner bearing surface (3) in a prepared acetabulum, comprising a handle (20), an impaction head (22) adapted to receive said cup (1), retention means (23) for attaching said cup (1) to the impaction head (22), and actuating means (24) for freeing the retention means (23) from the cup (1), **characterised in that** the handle carries an anvil (21), in which the actuating means (24) is in the handle (20) and is operatively connected to the anvil (21), in which the retention means (23) extends between the actuating means (24) and the impaction head (22), and in which impaction of the anvil (21) operates said actuating means (24) to free the retention means (23) from the cup (1).

2. An inserter as claimed in claim 1 in which the acetabular cup (1) has an opening (7) in its inner bearing surface (3) at least part of which extends adjacent to or over a substantially central part of the cup (1) and in which in a first position of said actuating means (24) the retention means (23) extends into the opening (7) in the inner bearing surface and engage the cup (1) to hold it in position on the impaction head (22), and in which said actuating means (24) can be operated to a second position to free said retention means (23) from the cup (1) by impacting the anvil (21).

3. An inserter as claimed in claim 1 or claim 2 in which the opening (7) in said inner bearing surface (3) extends through the wall of the cup (1) to its outer surface (6) to provide an outer rim (17).

4. An inserter as claimed in claim 1, claim 2 or claim 3 in which said actuating means (24) can also be operated to free the retaining means (23) independently from said anvil (21).

5. An inserter as claimed in claim 4, when dependent on claim 3, in which said retention means (23) include a retainer (25) which in the first position of the actuating means (24) is raised and engages the outer rim (17) of the opening (7) in the cup (1) and which in the second position the actuating means (24) is rotated and lowered relative to its first position and is clear of and lower than the outer rim (17) of the opening (7).

6. An inserter as claimed in claim 5 in which said retainer (25) is carried on a rotatable and axially movable elongate operating member (26) which extends between the impaction head (22) and the actuating means (24) in the handle (20).

7. An inserter as claimed in claim 6 including means (30) for resiliently biasing said actuating means (24) towards the first and second positions.

8. An inserter as claimed in claim 7 in which said resilient means (30) for resiliently biasing said actuating means (24) comprises a compression spring (30) which acts on said rotatable operating member (26).

9. An inserter as claimed in any one of the preceding claims 3 to 8 in which the retention means (23) includes a projecting finger or hook which in the first position of the actuating means (24) extends over or against the side of the opening (61) in the cup (60) and which in the second position of the actuating means is located within or below the opening (61) in the cup (60).

10. An inserter as claimed in any one of preceding claims 3 to 9 in which the actuating means (24) includes a control member (35) which projects radially from the side of the handle (20) and which is located in a control slot (37) within which it can move angularly to rotate the retention means (23), said slot (37) being shaped to cause the retention means (23) to also move axially when rotated.

11. An inserter as claimed in claim 10 in which said slot (37) has indentations (38)(39) at each end to locate said control member (35) in the first or second position of the actuating means (24).

12. An inserter as claimed in claim 10 or claim 11 in which said slot (38) is angled towards the end which corresponds to the second position of the actuating means (24).

13. An inserter as claimed in claims 1 to 10 in which said anvil (21) engages the end of the handle (20) when in the second position.

14. An inserter as claimed in claim 1 or claim 2 in which said opening (7) in said inner bearing surface (3) of the cup (60) is formed as a socket (61) with an inner slot (62) into which the retention means (23) extend to engage and hold the cup (1) in position on the impaction head (22).

15. An inserter as claimed in claim 1 in which said retention means (23) comprises two or more retention pads (74) which can be operated by the actuating means (24) to expand and grip the inner bearing surface (3) of the cup (70).

16. An inserter as claimed in claims 1 to 15 in which said impaction head (22) is readily removable from said handle (20).

17. An inserter as claimed in claim 16 which includes two or more impaction heads (22) of different configuration or size which can be fitted alternatively to said handle (20).

## Patentansprüche

1. Einsetzvorrichtung zum Positionieren und Einschlagen einer Hüftgelenkspfanne (1), die eine innere Lageroberfläche (3) aufweist, in einem vorbereiteten Acetabulum, umfassend einen Griff (20), einen Einschlagkopf (22), der angepasst ist, um die Pfanne (1) aufzunehmen, Haltemittel (23) zum Befestigen der Pfanne (1) am Einschlagkopf (22), und Betätigungsmittel (24) zum Freigeben der Haltemittel (23) von der Pfanne (1), **dadurch gekennzeichnet, dass** der Griff einen Amboss (21) trägt, bei dem sich das Betätigungsmittel (24) im Griff (20) befindet und mit dem Amboss (21) bewegend verbunden ist, bei dem sich das Haltemittel (23) zwischen dem Betätigungsmittel (24) und dem Einschlagkopf (22) erstreckt, und bei dem ein Einschlagen des Amboss (21) das Betätigungsmittel (24) bewegt, um das Haltemittel (23) von der Pfanne (1) freizugeben.

2. Einsetzvorrichtung nach Anspruch 1, bei der die Hüftgelenkspfanne (1) in ihrer inneren Lageroberfläche (3) eine Öffnung (7) aufweist, von der sich mindestens ein Teil benachbart zu oder über einen im Wesentlichen mittleren Teil der Pfanne (1) erstreckt, und bei der sich in einer ersten Stellung der Betätigungsmittel (24) das Haltemittel (23) in die Öffnung (7) in der inneren Lageroberfläche erstreckt und mit der Pfanne (1) in Eingriff tritt, um sie auf dem Einschlagkopf (22) in Position zu halten, und bei der das Betätigungsmittel (24) durch Einschlagen des Amboss (21) in eine zweite Stellung bewegt werden kann, um das Haltemittel (23) von der Pfanne (1) freizugeben.

3. Einsetzvorrichtung nach Anspruch 1 oder Anspruch 2, bei der sich die Öffnung (7) in der inneren Lageroberfläche (3) durch die Wand der Pfanne (1) bis zu ihrer äußeren Oberfläche (6) erstreckt, um einen Außenrand (17) bereitzustellen.

4. Einsetzvorrichtung nach Anspruch 1, Anspruch 2 oder Anspruch 3, bei der die Betätigungsmittel (24) auch unabhängig vom Amboss (21) bewegt werden können, um die Haltemittel (23) freizugeben.

5. Einsetzvorrichtung nach Anspruch 4, rückbezogen auf Anspruch 3, bei der die Haltemittel (23) einen Halter (25) einschließen, der in der ersten Stellung der Betätigungsmittel (24) angehoben ist und mit dem Außenrand (17) der Öffnung (7) in der Pfanne (1) in Eingriff tritt, und der in der zweiten Stellung der Betätigungsmittel (24) in Bezug zu seiner ersten Stellung gedreht und abgesenkt ist und frei von dem und tiefer als der Außenrand (17) der Öffnung (7) ist.

6. Einsetzvorrichtung nach Anspruch 5, bei der der Halter (25) auf einem drehbaren und axial beweglichen länglichen Betätigungselement (26) mitgeführt wird, das sich zwischen dem Einschlagkopf (22) und den Betätigungsmitteln (24) im Griff (20) erstreckt.

7. Einsetzvorrichtung nach Anspruch 6, einschließend Mittel (30) zum elastischen Vorspannen der Betätigungsmittel (24) in Richtung der ersten und zweiten Stellung.

8. Einsetzvorrichtung nach Anspruch 7, bei der die elastischen Mittel (30) zum elastischen vorspannen der Betätigungsmittel (24) eine Druckfeder (30) umfassen, die auf das drehbare Betätigungselement (26) einwirkt.

9. Einsetzvorrichtung nach einem der vorangehenden Ansprüche 3 bis 8, bei der das Haltemittel (23) einen überstehenden Finger oder Haken einschließt, der sich in der ersten Stellung der Betätigungsmittel (24) bis über oder gegen die Seite der Öffnung (61) in der Pfanne (60) erstreckt und der in der zweiten Stellung der Betätigungsmittel (24) in oder unterhalb von der Öffnung (61) in der Pfanne (60) angeordnet ist.

10. Einsetzvorrichtung nach einem der vorangehenden Ansprüche 3 bis 9, bei der das Betätigungsmittel (24) ein Steuerelement (35) einschließt, das aus der Seite des Griffs (20) radial übersteht und das in einem Steuerschlitz (37) angeordnet ist, in dem es sich um einen Winkel bewegen kann, um die Haltemittel (23) zu drehen, wobei der Schlitz (37) geformt ist, um zu bewirken, dass sich die Haltemittel (23) auch axial bewegen, wenn sie gedreht werden.

11. Einsetzvorrichtung nach Anspruch 10, bei der der Schlitz (37) an jedem Ende Einbuchtungen (38)(39) zum Positionieren des Steuerelements (35) in der ersten und zweiten Stellung der Betätigungsmittel (24) aufweist.

12. Einsetzvorrichtung nach Anspruch 10 oder Anspruch 11, bei der der Schlitz (38) zu dem Ende hin angewinkelt ist, das der zweiten Stellung der Betätigungsmittel (24) entspricht.

13. Einsetzvorrichtung nach den Ansprüchen 1 bis 10, bei der der Amboss (21) mit dem Ende des Griffs (20) im Eingriff steht, wenn er sich in der zweiten Stellung befindet.

14. Einsetzvorrichtung nach Anspruch 1 oder Anspruch 2, bei der die Öffnung (7) in der inneren Lageroberfläche (3) der Pfanne (60) als Büchse (61) mit einem inneren Schlitz (62) ausgebildet ist, in den sich die Haltemittel (23) erstrecken, um mit der Pfanne (1) in Eingriff zu treten und diese auf dem Einschlagkopf (22) in Position zu halten.

15. Einsetzvorrichtung nach Anspruch 1, bei der das Haltemittel (235 zwei oder mehr Haltekissen (74) umfasst, die durch die Betätigungsmittel (24) bewegt werden können, um sie aufzuweiten und die innere Lageroberfläche (3) der Pfanne (70) zu ergreifen.

16. Einsetzvorrichtung nach den Ansprüchen 1 bis 15, bei der der Einschlagkopf (22) mühelos vom Griff (20) abnehmbar ist.

17. Einsetzvorrichtung nach Anspruch 16, welche zwei oder mehr Einschlagköpfe (22) von unterschiedlicher Ausbildung oder Größe einschließt, die alternativ am Griff (20) angebracht werden können.

## Revendications

1. Dispositif d'insertion pour positionner et venir en impact avec un cotyle (1) qui comporte une surface de portée intérieure (3) dans un acétabule préparé, comprenant un manche (20), une tête d'impact (22) adaptée de façon à recevoir ledit cotyle (1), des moyens de maintien (23) pour fixer ledit cotyle (1) à la tête d'impact (22) et des moyens d'actionnement (24) pour libérer les moyens de maintien (23) vis-à-vis du cotyle (1), **caractérisé en ce que** le manche porte une enclume (21), les moyens d'actionnement (24) se trouvant dans le manche (20) et étant reliés de façon fonctionnelle à l'enclume (21), les moyens de maintien (23) s'étendant entre les moyens d'actionnement (24) et la tête d'impact (22), et l'impact de l'enclume (21) actionnant lesdits moyens d'actionnement (24) de façon à libérer les moyens de maintien (23) vis-à-vis du cotyle (1).

2. Dispositif d'insertion selon la revendication 1, dans lequel le cotyle (1) comporte une ouverture (7) dans sa surface de portée intérieure (3), dont au moins une partie s'étend au voisinage d'une partie sensiblement centrale du cotyle (1) ou sur celle-ci, et dans lequel, dans une première position desdits moyens d'actionnement (24), les moyens de maintien (23) s'étendent dans l'ouverture (7) dans la surface de portée intérieure et viennent en prise avec le cotyle (1) de façon à le maintenir en position sur la tête d'impact (22), et dans lequel lesdits moyens d'actionnement (24) peuvent être actionnés vers une deuxième position afin de libérer lesdits moyens de maintien vis-à-vis du cotyle (1) par impact de l'enclume (21).

3. Dispositif d'insertion selon la revendication 1 ou la revendication 2, dans lequel l'ouverture (7) dans ladite surface de portée intérieure (3) s'étend à travers la paroi du cotyle (1) jusqu'à sa surface extérieure (6) de façon à constituer un rebord extérieur (17).

4. Dispositif d'insertion selon la revendication 1, la revendication 2 ou la revendication 3, dans lequel lesdits moyens d'actionnement (24) peuvent également être actionnés de façon à libérer les moyens de maintien (23) indépendamment de ladite enclume (21).

5. Dispositif d'insertion selon la revendication 4, lorsqu'elle dépend de la revendication 3, dans lequel lesdits moyens de maintien (23) comprennent un élément de maintien (25) qui, dans la première position des moyens d'actionnement (24), est élevé et vient en prise avec le rebord extérieur (17) de l'ouverture (7) dans le cotyle (1), et qui, dans la deuxième position des moyens d'actionnement (24), est tourné et abaissé par rapport à sa première position, et est libéré du rebord extérieur (17) de l'ouverture (7) et est plus bas que celui-ci.

6. Dispositif d'insertion selon la revendication 5, dans lequel ledit élément de maintien (25) est porté sur un élément d'actionnement allongé rotatif et axialement mobile (26) qui s'étend entre la tête d'impact (22) et les moyens d'actionnement (24) dans le manche (20).

7. Dispositif d'insertion selon la revendication 6, comprenant des moyens (30) pour solliciter élastiquement lesdits moyens d'actionnement (24) vers les première et deuxième positions.

8. Dispositif d'insertion selon la revendication 7, dans lequel lesdits moyens élastiques (30) pour solliciter élastiquement lesdits moyens d'actionnement (24) comprennent un ressort de compression (30) qui agit sur ledit élément d'actionnement rotatif (26).

9. Dispositif d'insertion selon l'une quelconque des revendications 3 à 8 qui précédent, dans lequel les moyens de maintien (23) comprennent un crochet ou un doigt saillant, qui, dans la première position des moyens d'actionnement (24), s'étend sur ou contre le côté de l'ouverture (61) dans le cotyle (60), et qui, dans la deuxième position des moyens d'actionnement, est disposé à l'intérieur ou en dessous de l'ouverture (61) dans le cotyle (60).

10. Dispositif d'insertion selon l'une quelconque des revendications 3 à 9 qui précèdent, dans lequel les moyens d'actionnement (24) comprennent un élément de commande (35) qui fait saillie de façon radiale à partir du côté du manche (20), et qui est disposé dans une fente de commande (37) à l'intérieur de laquelle il peut se déplacer de façon angulaire de façon à faire tourner les moyens de maintien (23), ladite fente (37) étant formée de façon à amener les moyens de maintien (23) à se déplacer également de façon axiale lors de la rotation.

11. Dispositif d'insertion selon la revendication 10, dans lequel ladite fente (37) comporte des indentations (38) (39) à chaque extrémité de façon à positionner ledit élément de commande (35) dans la première ou la deuxième position des moyens d'actionnement (24).

12. Dispositif d'insertion selon la revendication 10 ou la revendication 11, dans lequel ladite fente (38) est en angle vers l'extrémité qui correspond à la deuxième position des moyens d'actionnement (24).

13. Dispositif d'insertion selon les revendications 1 à 10, dans lequel ladite enclume (21) vient en prise avec l'extrémité du manche (20) dans la deuxième position.

14. Dispositif d'insertion selon la revendication 1 ou la revendication 2, dans lequel ladite ouverture (7) dans ladite surface de portée inférieure (3) du cotyle (60) est formée sous la forme d'une douille (61) avec une fente intérieure (62) dans laquelle s'étendent les moyens de maintien (23) de façon à faire venir en prise et à maintenir le cotyle (1) en position sur la tête d'impact (22).

15. Dispositif d'insertion selon la revendication 1, dans lequel lesdits moyens de maintien (23) comprennent deux ou plusieurs tampons de maintien (74) qui peuvent être actionnés par les moyens d'actionnement (24) de façon à étendre et à saisir la surface de portée intérieure (3) du cotyle (70).

16. Dispositif d'insertion selon les revendications 1 à 10, dans lequel ladite tête d'impact (22) peut être facilement retirée dudit manche (20).

17. Dispositif d'insertion selon la revendication 16, qui comprend deux ou plusieurs têtes d'impact (22) d'une configuration ou d'une taille différente, qui peuvent être disposées en alternance sur ledit manche (20).
